# EUROPÄISCHE PATENTSCHRIFT

(11) **EP 0 920 276 B1**
(45) Veröffentlichungstag und Bekanntmachung des Hinweises auf die Patenterteilung: **20.08.2003**
(21) Anmeldenummer: 97937460.0
(22) Anmeldetag: 12.08.1997
(51) Int. Cl.: A61B 1/00, A61B 1/07, A61B 1/12

(54) **STARRES ENDOSKOP MIT BELEUCHTUNG**
RIGID ENDOSCOPE WITH LIGHTING
ENDOSCOPE RIGIDE AVEC ECLAIRAGE

(30) Priorität: 12.08.1996 DE 19632445; 18.09.1996 DE 19637963
(43) Veröffentlichungstag der Anmeldung: 09.06.1999
(73) Patentinhaber: MGB Endoskopische Geräte GmbH Berlin, 12489 Berlin (DE)
(72) Erfinder: BUESS, Gerhard, Fritz, D-72074 Tübingen (DE); FLEMMING, Ernst, D-82065 Baierbrunn (DE); KUNERT, Wolfgang, Jürgen, D-72074 Tübingen (DE); SCHURR, Marc, Oliver, D-72074 Tübingen (DE); NAUJOKS, Edward, D-12555 Berlin (DE); TSCHEPE, Johannes, D-10781 Berlin (DE)
(74) Vertreter: Kaden, Jutta
(86) Internationale Anmeldenummer: DE9701767
(87) Internationale Veröffentlichungsnummer: WO98006318

(56) Entgegenhaltungen:
- EP-A- 0 075 415
- EP-A- 0 647 425
- DE-B- 1 269 287
- GB-A- 2 197 496
- US-A- 4 727 416
- US-A- 5 101 468
- US-A- 5 533 496

## Beschreibung

Die vorliegende Erfindung betrifft ein starres Endoskop gemäß dem Oberbegriff des Anspruchs 1.

Es ist grundsätzlich bekannt, daß eine starre Optik für Endoskopie, im folgenden als starres Endoskop bezeichnet, aus einer für die Bildgebung verwendeten Optik und aus einer Lichtquelle zur Beleuchtung des betrachteten Objektes besteht. Es ist weiterhin beispielsweise aus der europäischen Patentschrift Nr. 0 369 937 A1 bekannt, daß die Lichtquelle mit einem parallel zur optischen Achse der Optik liegenden Glasfaserbündel, einem sogenannten Lichtwellenleiter, realisiert wird. In das Glasfaserbündel wird nach dem Stand der Technik am proximalen Ende (die dem Anwender zugewandte Seite) über ein weiteres Glasfaserbündel, das sogenannte Lichtleitkabel, das Licht einer externen Lichtquelle eingekoppelt. Über die Glasfaserbündel wird das Licht zum distalen Ende des Endoskops (die dem betrachteten Objekt zugewandte Seite) hin übertragen. Die Lichtquelle strahlt somit aus der Richtung des Betrachters auf das Objekt.

Derartige Endoskope weisen jedoch den Nachteil auf, daß chirurgische Instrumente, die sich während der Operation vor der Optik des Endoskops befinden, im allgemeinen keinen Schatten auf die betrachteten Organe werfen. Dies rührt daher, daß zwischen Beobachter und betrachtetem Objekt, hier im folgenden Bildhintergrund genannt, eingebrachte Gegenstände bekanntermaßen keine Schatten auf den Bildhintergrund werfen, wenn die Lichtaustrittsachse der Lichtquelle und die optische Bildachse des Beobachters zusammenfallen. Aufgrund des endoskopischen, zweidimensionalen Bildes fehlt dem Operateur ohnehin die Tiefeninformation über den betrachteten Raum, wodurch der räumliche Eindruck des Operationsbereiches eingeschränkt wird. Dieser räumliche Eindruck wird bei den genannten Endoskopen durch die unnatürliche Beleuchtung bzw. das Fehlen des Schattens noch weiter eingeschränkt.

Bei abgewinkelten Endoskopen, beispielsweise einem Laparoskop mit 30° Blickrichtung, ist konstruktionsbedingt eine kleine Asymmetrie zwischen der Lichtaustrittsachse der Lichtquelle und der optischen Bildachse des Beobachters vorhanden. Diese wird jedoch möglichst klein gehalten, so daß der Winkel zwischen der Lichtaustrittsachse der Lichtquelle und der optischen Bildachse des Beobachters gegen Null strebt und eine Schattenbildung, die die Plastizität der Bildwiedergabe erhöhen würde, vernachlässigbar klein ist.

Aus der deutschen Patentschrift 29 42 983 C2 ist ein Endoskop bekannt, bei dem zur Ausleuchtung des Nah- und des Fernbereiches vor der Optik des Endoskops zwei Lichtquellen vorgesehen sind. Beide Lichtquellen sind in Form von Lichtwellenleitern ausgebildet, deren freie Enden quer zur Längsrichtung des Endoskops unmittelbar neben dem distalen Ende der Endoskopoptik angeordnet sind. Die Lichtaustrittsachse der ersten Lichtquelle verläuft parallel zur Bildachse der Endoskopoptik und damit zur Blickrichtung des Beobachters. Um den Parallaxeneinfluß bei der Ausleuchtung des Nahbereiches durch die erste Lichtquelle auszuschalten, ist die Lichtaustrittsachse der zweiten Lichtquelle zur Bildachse der Endoskopoptik geneigt, wobei sich die Lichtaustrittsachse der zweiten Lichtquelle und die Bildachse der Endoskopoptik in Blickrichtung des Benutzers vor der Endoskopoptik schneiden. Durch die parallel zur Bildachse der Endoskopoptik strahlende erste Lichtquelle wird der Fembereich vor der Endoskopoptik und durch die geneigt zur Bildachse der Endoskopoptik strahlende zweite Lichtquelle wird der Nahbereich vor der Endoskopoptik ausgeleuchtet. Das Endoskop weist aber wie schon die oben genannten Endoskope infolge des geringen Abstandes zwischen dem distalen Ende der Endoskopoptik und den freien Enden der ersten und zweiten Lichtwellenleiter eine vernachlässigbar geringe Schattenbildung auf.

Die EP 0 647 425 zeigt ein Endoskop mit parallel zur Bildachse der Optik verlaufenden Glasfaserelementen und einem zusätzlichen Beleuchtungssystem, dass ebenfalls als Glasfaserbündel ausgebildet ist. Die Lichtaustrittsöffnung letzteren Glasfaserbündels liegt am distalen Ende der Optik.

Die EP 0 075 415 zeigt ein Endoskop, bei dem ein Teil eines am distalen Ende des Endoskops austretenden Lichtstrahls an einer Spiegelfläche reflektiert und auf die Oberfläche eines zu untersuchenden Objektes gelenkt wird.

Der Erfindung liegt daher die Aufgabe zugrunde, ein Endoskop zur Verfügung zu stellen, bei dem die Plastizität der Bildwiedergabe bei durch die Optik beobachteten dreidimensionalen Objekten, z. B. chirurgischen Instrumenten, erhöht ist.

Diese Aufgabe wird, ausgehend von einem starren Endoskop gemäß dem Oberbegriff des Anspruchs 1, durch die im kennzeichnenden Teil des Anspruchs 1 angegebenen Merkmale gelöst.

Die Erfindung schließt die technische Lehre ein, dass eine Vergrößerung der Plastizität der Bildwiedergabe bei dreidimensionalen Objekten erzielbar ist, wenn die Lichtaustrittsrichtung am Lichtaustritt des zweiten optischen Beleuchtungssystems in Bezug auf die Blickrichtung am distalen Ende der Optik, d. h. entlang der Bildachse der Optik seitlich um einen solchen Betrag versetzt ist, dass sich für Punkte auf einem betrachteten dreidimensionalen Objekt bezüglich des Lichtaustritts des zweiten optischen Beleuchtungssystems und des distalen Endes der Optik eine Parallaxe ergibt, die einen von Null verschiedenen Wert besitzt. Vorzugsweise handelt es sich bei dem optischen Beleuchtungssystem ebenfalls um ein Glasfaserbündel. Aufgrund dieser Parallaxe decken sich der Schatten, den das betrachtete dreidimensionale Objekt infolge des aus dem zweiten optischen Beleuchtungssystem austretenden Lichts auf den Bildhintergrund wirft, und die vom distalen Ende der Optik ausgehende Projektion des betrachteten auf den Bildhintergrund nicht. Der durch die Optik blickende Operateur nimmt folglich auf dem Bildhintergrund den Schatten des betrachteten dreidimensionalen Objekts wahr, wodurch die Plastizität der Bildwiedergabe in einfacher Weise erhöht wird. Diese Schattenbildung erleichtert dem Arzt die räumliche Orientierung in der endoskopierten Körperhöhle erheblich.

Ein weiterer Vorteil des erfindungsgemäßen Endoskops liegt darin, dass sich das Licht der beiden Glasfaserbündel im betrachteten Raum überlagert. Bezüglich des Lichtaustritts des ersten und des zweiten Glasfaserbündels entsteht ebenfalls eine von Null verschiedene Parallaxe, aufgrund derer eine ungleichmäßige Lichtverteilung im beobachteten Raum erzielt wird, die wiederum den Topographiekontrast auf dem betrachteten Objekt und auf dem Bildhintergrund erhöht und diese in vorteilhafter Weise für den Operateur somit ebenfalls plastischer in Erscheinung treten lässt. Zudem wird der betrachtete Raum diffuser bzw. gleichmäßiger ausgeleuchtet.

Der Lichtaustritt des zweiten Glasfaserbündels ist dabei in einem entsprechenden Abstand senkrecht zur Längsachse des Endoskops neben dem distalen Ende der Optik angeordnet. In Anbetracht des gering zu haltenden Endoskopdurchmessers ist die hierbei erzielbare Parallaxe und die dadurch bedingte Schattenbildung jedoch relativ begrenzt. Daher ist der Lichtaustritt des zweiten Glasfaserbündels um einen Abstand (d) vom distalen Ende der Optik zurückversetzt vorgesehen. Hierdurch ist auch bei geringem Endoskopdurchmesser eine relativ große Parallaxe und damit eine besonders plastische Bildwiedergabe erzielbar. Vorzugsweise beträgt der Abstand (d) etwa 5 bis 50 mm.

Bei vorteilhaften Ausführungen der Erfindung sind die Glasfasern des ersten und/oder des zweiten Glasfaserbündels über einen Winkelbereich verteilt koaxial um die Optik herum angeordnet. Hierdurch wird insbesondere bei zurückversetztem zweitem Glasfaserbündel eine Verschattung eines beträchtlichen Teiles des Beobachtungsraumes durch das distale Ende der Optik oder des Hüllrohres vermieden und so eine besonders günstige Ausleuchtung des Beobachtungsraumes erzielt. Vorzugsweise weisen das erste und/oder zweite Glasfaserbündel dabei einen sichelförmigen Querschnitt auf, da sich hierdurch eine besonders homogene Verteilung des aus dem jeweiligen Glasfaserbündel austretenden Lichts ergibt.

Bei besonders günstigen Weiterbildungen der Erfindung ist die Lichtaustrittsrichtung des zweiten Glasfaserbündels und/oder die Blickrichtung der Optik zur Bildachse der Optik bzw. zur Längsachse des Hüllrohrs geneigt ausgerichtet. Die Neigung der Lichtaustrittsrichtung des zweiten Glasfaserbündels zur Bildachse der Optik bewirkt dabei, daß die aus dem zweiten Glasfaserbündel austretenden Lichtstrahlen das von der Optik erfaßte Blickfeld im wesentlichen vollständig ausleuchten, wodurch eine entsprechende Schattenbildung über das von der Optik erfaßte Blickfeld sichergestellt ist.

Die Neigung der Blickrichtung der Optik, d. h. der optischen Bildachse der Optik, zur Längsachse des Endoskops bewirkt eine Neigung des gesamten durch die Optik erfaßten Blickfeldes. Hierdurch wird zum einen in einfacher Weise erreicht, daß die aus dem zweiten Glasfaserbündel austretenden, entsprechend eingestellten Lichtstrahlen das gesamte von der Optik erfaßte Blickfeld ausleuchten können, ohne daß ein wesentlicher Teil des Blickfeldes von dem sich zwischen dem Lichtaustritt des zweiten Glasfaserbündels und dem distalen Ende des Endoskops erstreckenden Teil des Endoskops verschattet wird. Zum anderen wird dadurch erreicht, daß die Parallaxe von Punkten im betrachteten Raum bezüglich des Lichtaustritts des zweiten Glasfaserbündels und des distalen Endes der Optik über weite Teile des durch die Optik erfaßten Blickfeldes einen von Null verschiedenen Wert besitzt, weshalb sich ein für den Operateur sichtbarer Schattenwurf ergibt.

Eine besonders gute Ausleuchtung und Schattenbildung im durch die Optik erfaßten Blickfeld ergibt sich insbesondere, wenn dabei die Lichtaustrittsrichtung des zweiten Glasfaserbündels und die Blickrichtung der Optik zur Längsachse des Hüllrohrs gleichsinnig geneigt ausgerichtet sind.

Vorzugsweise schließt die Lichtaustrittsrichtung des zweiten Glasfaserbündels mit der Blickrichtung der Optik einen

Winkel α größer Null ein, so daß die aus dem zweiten Glasfaserbündel austretenden Lichtstrahlen das durch die Optik erfaßte Blickfeld ausleuchten.

Bei bevorzugten Ausführungen des erfindungsgemäßen Endoskops ist das zweite Glasfaserbündel in das Hüllrohr integriert, wodurch sich in einfacher Weise ein für Beschädigungen der Beleuchtungseinrichtungen wenig anfälliges Endoskop ergibt.

Die Glasfasern des zweiten Glasfaserbündels weisen eine planar geschliffene, senkrecht zu deren Längsrichtung gerichtete Stirnfläche auf. Sie können aber auch in einem Winkel β zu einer solchen Stirnfläche geneigt angeschliffen sein. Hierdurch kann der Neigungswinkel der Lichtaustrittsrichtung zur Längsachse des Hüllrohrs bzw. deren Winkel α zur Bildachse der Optik in einfacher Weise eingestellt werden, ohne daß die Glasfasern selbst unter einem entsprechenden Winkel zur Längsachse des Hüllrohrs bzw. zur Bildachse der Optik angeordnet sein müssen.

Bei besonders günstigen Weiterbildungen des erfindungsgemäßen Endoskops ist der Lichtaustritt des zweiten, insbesondere planar geschliffenen, Glasfaserbündels längsverschieblich in Bezug auf das Hüllrohr angeordnet. Hierdurch kann in einfacher Weise der Abstand zwischen dem Lichtaustritt des zweiten Glasfaserbündels und dem distalen Ende der Optik variiert werden, wodurch sich auch die Parallaxe der Punkte im Betrachtungsraum hinsichtlich dieser beiden Bezugspunkte ändert, wodurch wiederum der vom Betrachter einsehbare Schattenwurf im Betrachtungsraum befindlicher Objekte eingestellt werden kann.

Bei bevorzugten Weiterbildungen des erfindungsgemäßen Endoskops sind die proximalen Enden des ersten und des zweiten Glasfaserbündel in einem Lichtleitkabelanschluß zusammengefaßt. Vorzugsweise ist die Anzahl der einzelnen Glasfasern des ersten und zweiten Glasfaserbündels dabei jeweils der zu übertragenden Lichtmenge angepaßt.

Bei bevorzugten Ausführungen des erfindungsgemäßen Endoskops ist in dem Hüllrohr mindestens ein Insufflationskanal oder mindestens ein Spülkanal, vorzugsweise beides, vorgesehen. Durch den Insufflationskanal kann z.B. CO₂-Gas in den Beobachtungsraum insuffliert werden und durch den Spülkanal eine isotonische Flüssigkeit. Der Insufflationskanal bzw. der Spülkanal ist vorzugsweise zwischen dem zweiten Glasfaserbündel und der Optik angeordnet ist, so daß hierdurch in einfacher Weise ein seitlicher Abstand zwischen der Lichtaustrittsrichtung am Lichtaustritt des zweiten Glasfaserbündels in Bezug auf die Blickrichtung am distalen Ende der Optik erzielt ist, der alleine schon eine für die erwünschte Schattenwirkung erforderliche Parallaxe sicherstellt.

Vorzugsweise ist an den jeweiligen distalen Enden des Insufflationskanals und/oder des Spülkanals ein Mittel zur Strahlformung angebracht, so daß mit Hilfe des Gasstromes und/oder des Flüssigkeitsstromes das distale Ende des Endoskops und insbesondere die Bildeintrittsfläche reinigbar ist. Ein Einsatz des Endoskops, beispielsweise bei chirurgischen Eingriffen mit energetischen Therapiegeräten, z.B. Hochfrequenz-Chirurgie oder Laser-Chirurgie, bei dem das distale Ende der Endoskope, insbesondere die Bildeintrittsfläche der Optik, verunreinigt wird ist somit ohne störende Unterbrechungen zur- Reinigung der Bildeintrittsfläche der Optik möglich.

Bei Temperaturunterschieden zwischen einer Optik und deren Umgebung gibt es einen von dem Sättigungsgrad, d. h. der relativen Luftfeuchtigkeit, abhängigen Feuchtigkeitsniederschlag an der Optik. Im hier betrachteten Anwendungsfall der Endoskopie ist die Optikumgebung zu 90 bis 100% gesättigtes CO₂-Gas bei Körpertemperatur, so daß die distale Bildeintrittsfläche der Optik gemäß der Taupunktkurve abhängig von ihrer aktuellen Temperatur mehr oder weniger stark beschlägt. In konsequenter Weiterführung des Erfindungsgedankens ist in dem Hüllrohr eine Heizung vorgesehen, die vorzugsweise das Insufflationsgas, die Spülflüssigkeit und/oder das Hüllrohr einschließlich mindestens eines der Glasfaserbündel auf Körpertemperatur temperiert und so ein Beschlagen der distalen Bildeintrittsfläche der Optik verhindert. Alternativ wird durch Temperieren der Optik und/oder den auf die Bildeintrittsfläche gerichteten temperierten Gasstrom ein Beschlagen der distalen Bildeintrittsfläche der Optik herabgesetzt ist.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Seitenansicht eines bevorzugten Ausführungsbeispiels der Erfindung,
- Figur 2: eine Seitenansicht eines weiteren bevorzugten Ausführungsbeispiels der Erfindung,
- Figur 2a: eine schematische Schnittansicht des Lichtleitkabelanschlusses aus Figur 2,
- Figur 3: eine Draufsicht auf das distale Ende eines erfindungsgemäßen Endoskops,
- Figur 4: einen Schnitt durch das distale Ende des Endoskops aus Figur 3 entlang der Linie IV-IV.

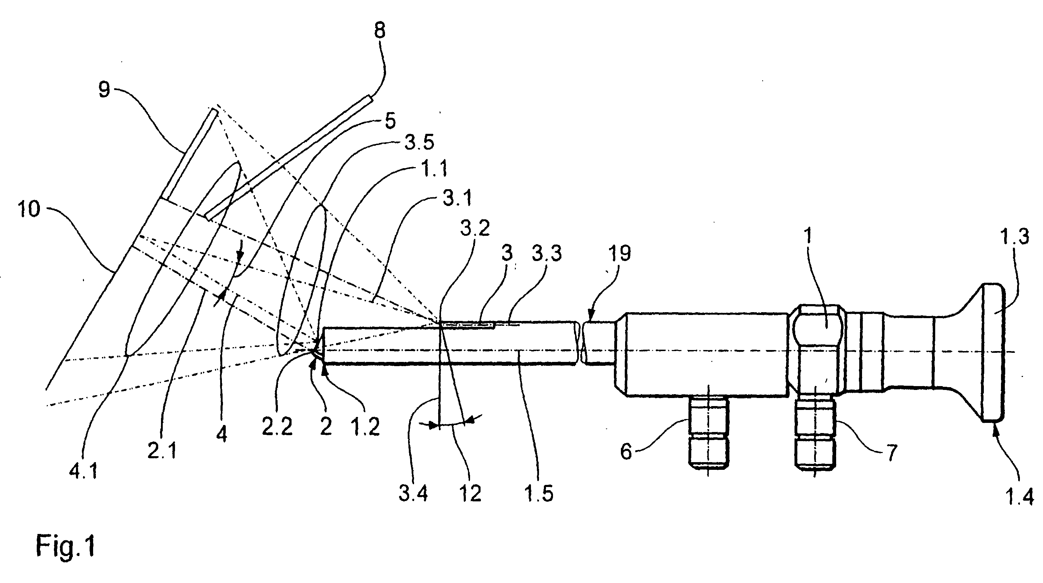

Figur 1 zeigt ein langgestrecktes, starres Endoskop mit einer in einem Hüllrohr 19 integrierten Optik 1, dem ersten Glasfaserbündel 2 und dem zweiten Glasfaserbündel 3. Die Optik 1 umfaßt dabei ein Objektiv 1.1, das am distalen Ende 1.2 des Endoskops, d. h. am dem zu betrachtenden Objekt zugewandten Ende des Endoskops angebracht ist, und ein Okular 1.3, das sich an dem proximalen Ende 1.4 des Endoskops, d.h. am dem Operateur zugewandten Ende des Endoskops befindet.

Das Objektiv 1.1 ist so am distalen Ende 1.2 des Endoskops angeordnet, daß die optische Bildachse 4 des Objektivs 1.1 - und damit der Optik 1 - zur Längsachse 1.5 des Endoskops um einen Winkel von ca. 30° geneigt verläuft. Die weist dabei eine zur Längsachse 1.5 senkrechte Verlaufskomponente auf, die in Richtung der Seite des Endoskops weist, an der das zweite Glasfaserbündel 3 angeordnet ist. Der in Blickrichtung des Operateurs vom Objektiv 1.1 ausgehende Teil der optischen Bildachse 4 ist somit zur Längsachse 1.5 des Endoskops um den genannten Winkel in Richtung der Seite des Endoskops geneigt, an der das zweite Glasfaserbündel 3 angeordnet ist.

Der Lichtaustritt 2.2 am distalen Ende des ersten Glasfaserbündels 2 ist ebenfalls am distalen Ende 1.2 des Endoskops angeordnet, wobei das erste Glasfaserbündel 2 so ausgerichtet ist, daß seine Lichtaustrittsachse 2.1 parallel zur Bildachse 4 des Objektivs 1.1 verläuft. Das zweite Glasfaserbündel 3 ist am Endoskop in Längsrichtung des Endoskops von dessen distalem Ende 1.2 zurückversetzt angeordnet. In Längsrichtung des Endoskops entspricht der Abstand (d) des Lichtaustritts 3.2 am distalen Ende des Glasfaserbündels 3 vom distalen Ende 1.2 des Endoskops im gezeigten Beispiel etwa dem dreifachen Durchmesser des Endoskops an seinem distalen Ende 1.2. Je nach Durchmesser des Endoskops beträgt der Abstand (d) zwischen 5 und 50 mm. -Die Lichtaustrittsachse 3.1 des zweiten Glasfaserbündels 3 ist unter einem Winkel 5 (also dem Winkel α) zur Bildachse 4 des Objektivs 1.1 geneigt, der bei dem dargestellten Ausführungsbeispiel etwa 15° beträgt. Es versteht sich, daß der Winkel 5 - entsprechend den vorliegenden Voraussetzungen - auch anders gewählt sein kann.

Um den Winkel 5 der Lichtaustrittsachse 3.1 des zweiten Glasfaserbündels 3 zur Bildachse 4 des Objektivs 1.1 einzustellen, sind die Glasfasern des zweiten Glasfaserbündels 3 am Lichtaustritt 3.2 abgeschrägt ausgebildet. Die Stirnfläche der Glasfasern am Lichtaustritt 3.2 ist dabei unter einem Winkel 12 (also dem Winkel β) von - beim vorliegenden Ausführungsbeispiel - etwa 13° zur Normalebene 3.4 auf der Längsachse 3.3 des zweiten Glasfaserbündels 3 geneigt, wobei die Stirnfläche dem Endoskop zugewandt ist. Hierdurch weist die Lichtaustrittsachse 3.1 des zweiten Glasfaserbündels 3 in Lichtaustrittsrichtung vom Endoskop weg. Um die Abschrägung am distalen Ende 3.2 des zweiten Glasfaserbündels 3 zu erzielen, sind die Glasfasern entsprechend planar geschliffen. Die Winkel 5 und 12 (also α und β) stehen in einem direkten Zusammenhang.

Das zweite Glasfaserbündel 3 ist in Längsrichtung des Endoskops verschieblich am Endoskop angeordnet. Hierdurch kann der einem Abstand des Lichtaustritts 3.2 des zweiten Glasfaserbündels 3 vom distalen Ende 1.2 des Endoskops und damit dem distalen Ende des Objektivs 1.1 variiert werden. Durch sinnvolle Wahl dieses Abstandes wird die Schattenwirkung kontrastreich eingestellt. Es versteht sich jedoch, daß bei weiteren vorteilhaften Ausbildungen der Erfindung die Glasfasern des zweiten Glasfaserbündels 3 nicht mit einem Winkel 12 angeschliffen, sondern mit einer zu ihrer Längsachse senkrechten Endfläche ausgebildet sein können. Hierbei kann die Schattenwirkung dann ebenfalls durch die axiale Entfernung des distalen Endes 3.2 des zweiten Glasfaserbündels 3 zur Bildachse 4 eingestellt werden.

Das Licht für die Glasfaserbündel 2 und 3 wird getrennt in die jeweiligen Lichtleiterkabelanschlüsse 6 und 7 eingekoppelt. Das Licht der beiden Glasfaserbündel 2 und 3 überlagert sich im betrachteten Raum. Ein in den Betrachtungsraum eingebrachter Gegenstand 8 wirft bedingt durch das aus dem distalen Ende 3.2 des zweiten Glasfaserbündels 3 austretende Licht einen Schatten 9 auf den Bildhintergrund 10. Aufgrund des Abstandes zwischen dem am distalen Ende 1.2 angeordneten Objektiv 1.1 und dem Lichtaustritt 3.2 des zweiten Glasfaserbündels 3 decken sich der Schatten 9 und die Projektion des Gegenstandes 8 auf den Bildhintergrund 10 ausgehend von der distalen Endfläche des Objektivs 1.1 nicht. Der durch das Okular 1.3 blickende Operateur nimmt folglich auf dem Bildhintergrund 10 den Schatten des Gegenstandes 8 wahr.

Der Abstand zwischen den Lichtaustritten 2.2 und 3.2 der beiden Glasfaserbündel 2 und 3 und ihre zueinander geneigt verlaufenden Lichtaustrittsrichtungen 2.1 und 3.1 bewirken im übrigen, daß der betrachtete Raum in vorteilhafter Weise diffuser bzw. gleichmäßiger ausgeleuchtet wird und der Topographiekontrast auf dem Bildhintergrund 10 aber auch auf dem betrachteten Gegenstand 8 erhöht wird, wodurch dem Operateur infolge der erhöhten Plastizität der Bildwiedergabe das Arbeiten wesentlich erleichtert wird.

Die Neigung des in Blickrichtung des Operateurs vom Objektiv 1.1 ausgehenden Teiles der optischen Bildachse 4 zur Längsachse 1.5 des Endoskops in Richtung des zweiten Glasfaserbündels 3 bewirkt eine Neigung des gesamten durch das Objektiv 1.1 erfaßten Blickfeldes 4.1 zur Längsachse des Endoskops. Hierdurch wird zum einen in einfacher Weise erreicht, daß der aus dem zweiten Glasfaserbündel 3 austretende, gleichsinnig mit der optischen Bildachse 4 zur Längsachse 1.5 geneigte Lichtkegel 3.5 - bis zu einer bestimmten Maximalentfernung des Endoskops vom Bildhintergrund 10 - das gesamte Blickfeld 4.1 ausleuchtet, ohne daß ein wesentlicher Teil des Blickfeldes 4.1 von dem sich zwischen dem Lichtaustritt 3.2 des zweiten Glasfaserbündels 3 und dem distalen Ende 1.2 des Endoskops erstreckenden Teil des Endoskops verschattet wird. Zum anderen wird dadurch erreicht, daß die Parallaxe von Punkten auf dem Gegenstand 8 bezüglich des Lichtaustritts 3.2 des zweiten Glasfaserbündels 3 und der distalen Endfläche des Objektivs 1.1 über weite Teile des Blickfeldes 4.1 einen von Null verschiedenen Wert besitzt, weshalb sich ein für den Operateur sichtbarer Schattenwurf auf den Bildhintergrund 10 ergibt. Lediglich in dem kleinen Randbereich des Blickfeldes 4.1, in dem die Mantellinien der kegelförmigen Begrenzung des Blickfeldes 4.1 etwa parallel zur Längsachse des Endoskops verlaufen, verschwindet der Schatten 9 für den Betrachter mehr oder weniger hinter dem Gegenstand 8, da in diesem Bereich die genannte Parallaxe gegen Null geht.

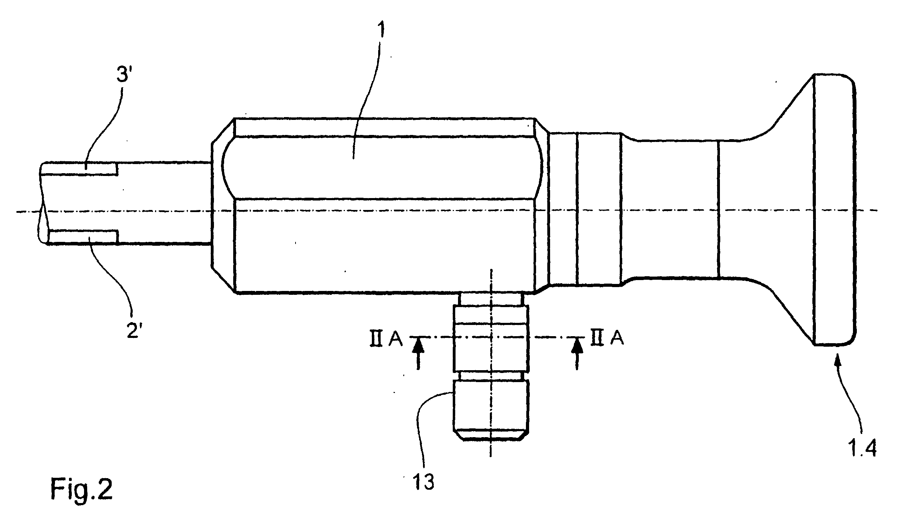

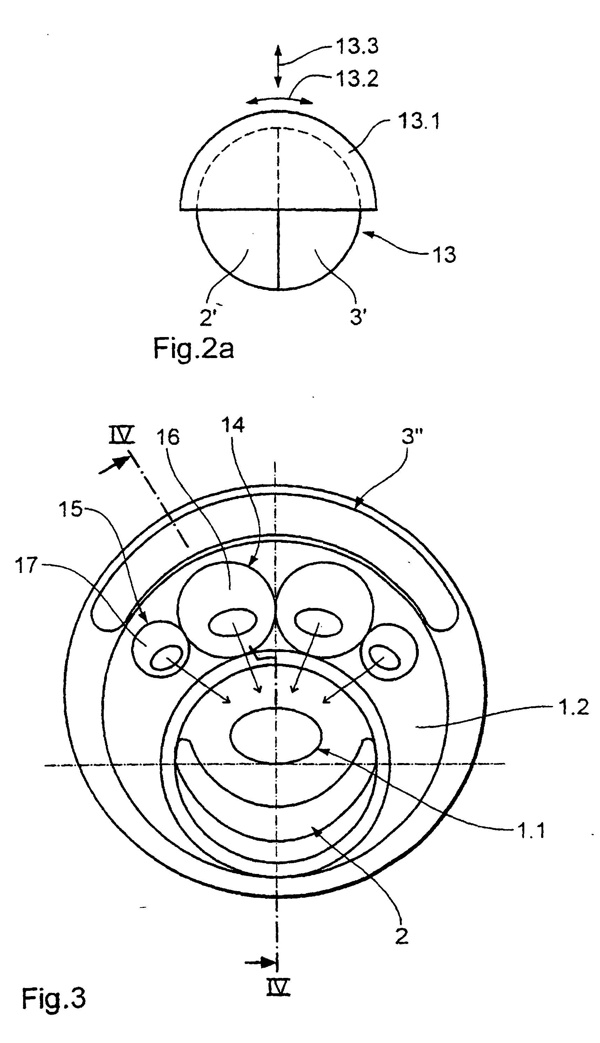

In Figur 2 ist das proximale Ende 1.4 eines Endoskops dargestellt, das im wesentlichen dem Endoskop aus Figur 1 entspricht. Der Unterschied zum Endoskop aus Figur 1 besteht darin, daß die Glasfaserbündel 2' und 3' in einem gemeinsamen Lichtleitkabelanschluß 13 zusammengefaßt sind. Figur 2a zeigt eine schematische Schnittansicht des Lichtleitkabelanschlusses 13 entlang Linie IIA-IIA aus Figur 2. Der Lichtleitkabelanschluß 13 ist mit einer Blende 13.1 versehen, die den Querschnitt der proximalen Enden der beiden Glasfaserbündel 2' und 3' mehr oder weniger stark abdeckt. Diese Blende 13.1 kann in ihrer Ebene entlang des Pfeiles 13.2 verschoben werden, um den Querschnitt der proximalen Enden der beiden Glasfaserbündel 2' und 3' mehr oder weniger stark abzudecken und so die in die proximalen Enden des ersten und des zweiten Glasfaserbündels 2' und 3' eingekoppelte Lichtmenge einzustellen. Zum Einstellen der Verteilung der insgesamt eingekoppelten Lichtmenge auf das erste und das zweite Glasfaserbündel 2' und 3' ist die Blende 13.1 in ihrer Ebene entlang des Pfeiles 13.3 um eine zur Zeichenebene senkrechte Achse drehbar. Hierdurch kann das Verhältnis zwischen der Abdeckung des Querschnitts des proximalen Endes des ersten Glasfaserbündels 2' und der Abdeckung des Querschnitts des proximalen Endes des zweiten Glasfaserbündels 3' durch die Blende 13.1 eingestellt werden, so daß die Verteilung der insgesamt im Lichtleitkabelanschluß 13 eingekoppelten Lichtmenge auf die beiden Glasfaserbündel 2' und 3' eingestellt werden kann. Es versteht sich, daß eine derartige Einstelleinrichtung, insbesondere eine Blende, und deren Verstellbarkeit aber in vielfacher anderer bekannter Weise realisiert sein kann sein kann.

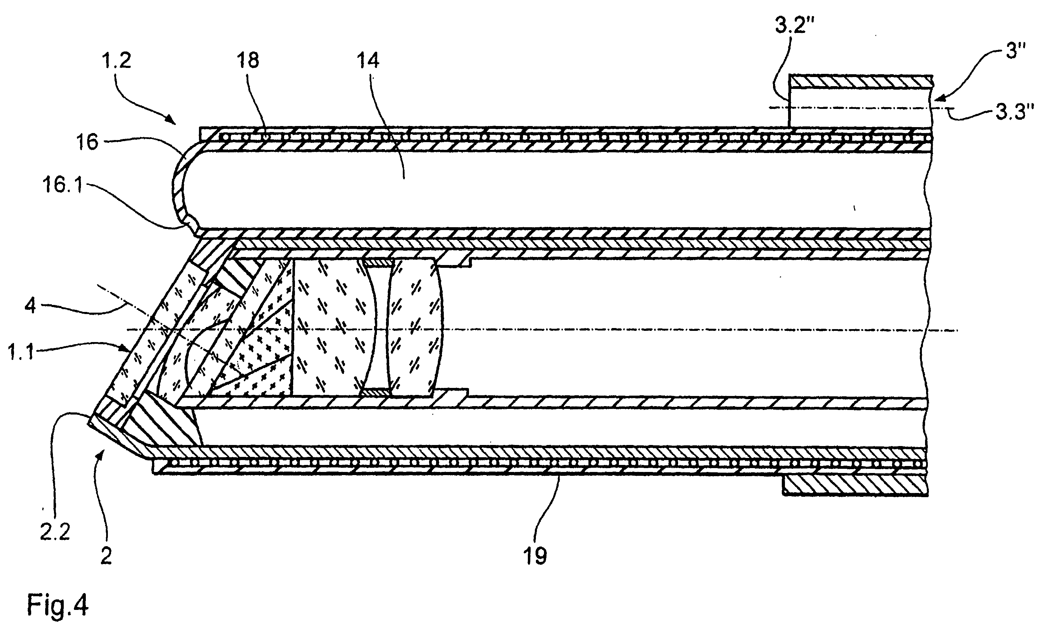

Figur 3 zeigt eine Draufsicht auf das distale Ende 1.2 eines Endoskops, das im wesentlichen dem Endoskop aus Figur 1 entspricht, Figur 4 zeigt einen Schnitt durch das distale Ende des Endoskops aus Figur 3 entlang der Linie IV-IV. Der Unterschied zum Endoskop aus Figur 1 besteht darin, daß die Endfläche am distalen Ende 3.2" des zweiten Glasfaserbündels 3" senkrecht auf der Längsachse 3.3" des zweiten Glasfaserbündels 3" steht, weshalb die Längsachse 3.3" auch die Lichtaustrittsachse des zweiten Glasfaserbündels 3" bildet.

Wie aus Figur 3 ersichtlich ist, sind die Glasfasern des ersten Glasfaserbündels 2 über einen Winkelbereich von etwa 180° und die Glasfasern des zweiten Glasfaserbündels 3 über einen Winkelbereich von etwa 120° koaxial um die Optik 1 herum angeordnet. Das erste und zweite Glasfaserbündel 2 und 3 weisen dabei einen sichelförmigen Querschnitt auf.

Weiterhin sind bei dieser Ausführung der Erfindung Insufflationskanäle 14 und Spülkanäle 15 in dem Endoskop integriert, die zwischen dem zweiten Glasfaserbündel 3 und der Optik 1 angeordnet sind. Durch den Insufflationskanal 14 kann z.B. CO₂-Gas insuffliert werden, während durch den Spülkanal isotonische Spülflüssigkeit gepumpt wird. Die jeweiligen distalen Enden der Insufflationskanäle 14 und der Spülkanäle 15 sind mit Mitteln 16 und 17 zur Strahlformung abgeschlossen. Wie Figur 4 zu entnehmen ist, bestehen die an den Insufflationskanälen 14 angeformten Mittel zur Strahlformung aus einer etwa halbkugelschalenförmigen Kappe 16, die den jeweiligen Kanal 14 abschließt. Diese Kappe 16 ist mit einer Öffnung 16.1 versehen, die den Insufflationsgasstrom auf das Objektiv 1.1 lenkt. Mit Hilfe des Gasstromes und des Spülflüssigkeitsstromes wird das distale Ende 1.2 des Endoskops und insbesondere die Bildeintrittsfläche des Objektivs 1.1 gereinigt. Hierdurch wird verhindert, daß bei chirurgischen Eingriffen mit energetischen Therapiegeräten, z.B. Hochfrequenz-Chirurgie oder Laser-Chirurgie, das distale Ende 1.2 des Endoskops, insbesondere das Objektiv 1.1 bleibend verunreinigt wird und ein Weiterarbeiten erschwert bzw. ganz unmöglich macht.

Bei Temperaturunterschieden zwischen der Optik und deren Umgebung entsteht ein von dem Sättigungsgrad, d.h. der relativen Luftfeuchtigkeit abhängiger Feuchtigkeitsniederschlag an der Optik. Im hier betrachteten Anwendungsfall der Endoskopie ist die Optikumgebung zu 90 bis 100% gesättigtes CO₂-Gas bei Körpertemperatur, so daß die distale Bildeintrittsfläche des Objektivs 1.1 gemäß der Taupunktkurve abhängig von ihrer aktuellen Temperatur mehr oder weniger stark beschlägt. In konsequenter Weiterführung des Erfindungsgedankens wird ist daher in das Endoskop eine Heizung derart integriert, daß das Insufflationsgas, die Spülflüssigkeit und das Objektiv 1.1 auf Körpertemperatur gehalten werden und ein Beschlagen des Objektivs 1.1, d. h. der distalen Bildeintrittsfläche der Optik 1 vermieden wird. Die Heizung besteht im gezeigten Beispiel aus einer elektrischen Heizwendel 18, die über die Länge des Endoskops im Hüllrohr 19 des Endoskops eingebettet ist und das gesamte Endoskop auf Körpertemperatur hält.

## Patentansprüche

1. Starres Endoskop bestehend aus einem Hüllrohr (19) mit einer Optik (1) und einem parallel zur Bildachse (4) der Optik (1) strahlenden ersten Glasfaserbündel (2, 2') und einem zweiten optischen Beleuchtungssystem, wobei das erste Glasfaserbündel (2, 2') und das zweite optische Beleuchtungssystem derart angeordnet sind, dass sie ein von der Optik (1) erfasstes Blickfeld ausleuchten, und wobei die Lichtaustrittsrichtung (3.1) am Lichtaustritt (3.2, 3.2', 3.2") des zweiten optischen Beleuchtungssystem in Bezug auf die Blickrichtung entsprechend der Bildachse (4) am distalen Ende (1.2) der Optik (1) seitlich um einen solchen Betrag versetzt ist, dass aufgrund der sich ergebenden Parallaxe und der damit erzielten Schattenbildung bzw. ungleichmäßigen Lichtverteilung die Plastizität der Bildwiedergabe bei dreidimensionalen Objekten (8) vergrößert ist, **dadurch gekennzeichnet, dass** das zweite optische Beleuchtungssystem um einen Abstand (d) im Bereich von 5 bis 50 mm vom distalen Ende (1,2) der Optik (1) zurückversetzt ist.

2. Endoskop nach Anspruch 1, **dadurch gekennzeichnet, dass** es sich bei dem zweiten optischen Beleuchtungssystem um ein Glasfaserbündel (3, 3', 3") handelt.

3. Endoskop nach Anspruch 2, **dadurch gekennzeichnet, dass** die Glasfasern des ersten und/oder zweiten Glasfaserbündels (2, 2', 3, 3', 3") über einen Winkelbereich verteilt koaxial um die Optik (1) herum angeordnet sind.

4. Endoskop nach Anspruch 3, **dadurch gekennzeichnet, dass** das erste und/oder zweite Glasfaserbündel (2, 2', 3, 3', 3") einen sichelförmigen Querschnitt aufweist bzw. aufweisen.

5. Endoskop nach Anspruch 2, **dadurch gekennzeichnet, dass** die Lichtaustrittsrichtung (3.1) des zweiten Glasfaserbündels (3, 3', 3") und die Blickrichtung der Optik (1) zur Längsachse (1.5) des Hüllrohrs (19) geneigt ausgerichtet sind.

6. Endoskop nach Anspruch 5, **dadurch gekennzeichnet, dass** die Neigung der Lichtaustrittsrichtung (3.1) des zweiten Glasfaserbündels (3, 3', 3") und die Blickrichtung der Optik (1) gleichsinnig ist.

7. Endoskop nach den Ansprüchen 5 oder 6, **dadurch gekennzeichnet, dass** die Lichtaustrittsrichtung (3.1) des zweiten Glasfaserbündels (3, 3', 3") mit Blickrichtung (4) der Optik (1) einen Winkel α (5) größer Null einschließt.

8. Endoskop nach Anspruch 2, **dadurch gekennzeichnet, dass** das zweite Glasfaserbündel (3, 3', 3") in das Hüllrohr (19) integriert ist.

9. Endoskop nach Anspruch 2, **dadurch gekennzeichnet, dass** sowohl die Lichtaustritte (2.2, 3.2, 3.2', 3.2") des ersten und zweiten Glasfaserbündels (2, 2', 3, 3', 3") als auch der objektseitige Teil (1.1) der Optik (1) im Bereich des distalen Endes (1.2) des Endoskops angeordnet sind.

10. Endoskop nach Anspruch 2, **dadurch gekennzeichnet, dass** die Glasfasern im Lichtaustritt (3.2, 3.2', 3.2") des zweiten Glasfaserbündels (3, 3', 3") eine planar geschliffene, senkrecht zu ihrer Längsrichtung gerichtete Stirnfläche aufweisen oder dass sie in einem Winkel β (12) zu einer solchen Stirnfläche geneigt angeschliffen sind.

11. Endoskop nach Anspruch 2, **dadurch gekennzeichnet, dass** der Lichtaustritt (3.2, 3.2', 3.2") des zweiten, insbesondere planar geschliffenen, Glasfaserbündels (3, 3', 3") zur Beeinflussung der Schattenwirkung längsverschieblich in Bezug auf das Hüllrohr (19) angeordnet ist.

12. Endoskop nach Anspruch 2, **dadurch gekennzeichnet, dass** die proximalen Enden des ersten und des zweiten Glasfaserbündels (2, 2', 3, 3', 3") in einem gemeinsamen Lichtleitkabelanschluss (13) zusammengefasst sind.

13. Endoskop nach Anspruch 12, **dadurch gekennzeichnet, dass** der gemeinsamen Lichtleitkabelanschluss (13) eine Einrichtung (13.1), insbesondere eine Blende, zum Einstellen der in die proximalen Enden des ersten und des zweiten Glasfaserbündels (2, 2', 3, 3', 3") eingekoppelten Lichtmenge und/oder zum Einstellen der Verteilung der eingekoppelten Lichtmenge auf das erste und das zweite Glasfaserbündel (2, 2', 3, 3', 3") umfasst.

14. Endoskop nach Anspruch 2, insbesondere nach Anspruch 12, **dadurch gekennzeichnet, dass** die Anzahl der einzelnen Glasfasem des ersten und zweiten Glasfaserbündels (2, 2', 3, 3', 3") jeweils der zu übertragenden Lichtmenge angepasst ist.

15. Endoskop nach Anspruch 1, **dadurch gekennzeichnet, dass** in dem Hüllrohr (19) mindestens ein Insufflationskanal (14) und/oder mindestens ein Spülkanal (15) vorgesehen ist bzw. sind.

16. Endoskop nach den Ansprüchen 2 und 15, **dadurch gekennzeichnet, dass** der Insufflationskanal (14) zwischen dem zweiten Glasfaserbündel (3, 3', 3") und der Optik (1) angeordnet ist.

17. Endoskop nach Anspruch 15, **dadurch gekennzeichnet, dass** am distalen Ende des Insufflationskanal (14) ein Mittel (16, 17) zur Strahlformung angebracht ist, das zur Reinigung des distalen Endes (1.2) des Endoskops und insbesondere der Bildeintrittsfläche dient.

18. Endoskop nach Anspruch 1, **dadurch gekennzeichnet, dass** in dem Hüllrohr (19) eine Heizung (18) zur Temperierung eines im Insufflationskanal (14) geförderten Insufflationsgases vorgesehen ist.

19. Endoskop nach Anspruch 1, **dadurch gekennzeichnet, dass** im Hüllrohr (19) eine Heizung (18) zur Temperierung einer im Spülkanal (15) geförderten Spülflüssigkeit vorgesehen ist.

20. Endoskop nach Anspruch 2, **dadurch gekennzeichnet, dass** im Hüllrohr (19) eine Heizung (18) zur Temperierung des Hüllrohrs (19) einschließlich mindestens eines der Glasfaserbündel (2, 2', 3, 3', 3") vorgesehen ist.

21. Endoskop nach Anspruch 15, **dadurch gekennzeichnet, dass** am distalen Ende des Spülkanals (15) ein Mittel (16, 17) zur Strahlformung angebracht ist, das zur Reinigung des distalen Endes (1.2) des Endoskops und insbesondere der Bildeintrittsfläche dient.

22. Endoskop nach den Ansprüchen 2 und 15, **dadurch gekennzeichnet, dass** der Spülkanal (15) zwischen dem zweiten Glasfaserbündel (3, 3', 3") und der Optik (1) angeordnet ist.

## Claims

1. Rigid endoscope consisting of a jacket tube (19) having an optical system (1) and a first glass fibre bundle (2, 2'), radiating parallel to the image axis (4) of the optical system (1), and a second optical illumination system, the first glass fibre bundle (2, 2") and the second optical illumination system being arranged in such a way that they illuminate a field of vision captured by the optical system (1) and the light exit direction (3.1) at the light exit (3.2, 3.2', 3.2") of the second optical illumination system being offset laterally relative to the viewing direction in accordance with the image axis (4) on the distal end (1.2) of the optical system (1) by an amount such that because of the resultant parallax and the shadow formation and unequal light distribution thus attained, the plasticity of the image reproduction for three-dimensional objects (8) is increased, **characterised in that** the second optical illumination system is set back by a distance (d) in the range of 5 to 50 mm from the distal end (1.2) of the optical system (1).

2. Endoscope according to claim 1, **characterised in that** the second optical illumination system is a glass fibre bundle (3, 3', 3").

3. Endoscope according to claim 2, **characterised in that** the glass fibres of the first and/or second glass fibre bundle (2, 2', 3, 3', 3") are distributed over an angular region coaxially about the optical system (1).

4. Endoscope according to claim 3, **characterised in that** the first and/or second glass fibre bundle (2, 2', 3, 3', 3") has/have a crescent-shaped cross-section.

5. Endoscope according to claim 2, **characterised in that** the light exit direction (3.1) of the second glass fibre bundle (3, 3', 3") and the viewing direction of the optical system (1) is oriented at an inclination to the longitudinal axis (1.5) of the jacket tube (19).

6. Endoscope according to claim 5, **characterised in that** the inclination of the light exit direction (3.1) of the second glass fibre bundle (3, 3', 3") and the viewing direction of the optical system (1) are in the same direction.

7. Endoscope according to claim 5 or 6, **characterised in that** the light exit direction (3.1) of the second glass fibre bundle (3, 3', 3") forms an angle α (5) of greater than zero with the viewing direction (4) of the optical system (1).

8. Endoscope according to claim 2, **characterised in that** the second glass fibre bundle (3, 3', 3") is integrated with the jacket tube (19).

9. Endoscope according to claim 2, **characterised in that** both the light exits (2.2, 3.2, 3.2', 3.2,") of the first and second glass fibre bundles (2, 2', 3, 3', 3") and the portion (1.1) of the optical system (1) toward the object are arranged in the region of the distal end (1.2) of the endoscope.

10. Endoscope according to claim 2, **characterised in that** the glass fibres at the light exit (3.2, 3.2', 3.2") of the second glass fibre bundle (3, 3', 3") have an end face that is ground plane and is oriented perpendicular to their longitudinal direction, or that they are ground so as to be inclined by an angle β (12) to such an end face.

11. Endoscope according to claim 2, **characterised in that** the light exit (3.2, 3.2', 3.2") of the second, in particular plane-ground, glass fibre bundle (3, 3', 3") is arranged longitudinally displaceably with respect to the jacket tube (19), in order to influence the shadow effect.

12. Endoscope according to claim 2, **characterised in that** the proximal ends of the first and second glass fibre bundle (2, 2', 3, 3', 3") are joined together in a common optical fibre cable connection (13).

13. Endoscope according to claim 12, **characterised in that** the common optical fibre cable connection (13) includes a device (13.1), in particular an aperture, for adjusting the quantity of light input into the proximal ends of the first and second glass fibre bundles (2, 2', 3, 3', 3") and/or adjusting the distribution of the quantity of light input to the first and second glass fibre bundles (2, 2', 3, 3', 3").

14. Endoscope according to claim 2, in particular according to claim 12, **characterised in that** the number of individual glass fibres in the first and second glass fibre bundles (2, 2', 3, 3', 3") are adapted per bundle to the quantity of light to be transmitted.

15. Endoscope according to claim 1, **characterised in that** at least one insufflation channel (14) and/or at least one flushing channel (15) is/are provided in the jacket tube (19).

16. Endoscope according to claims 2 and 15, **characterised in that** the insufflation channel (14) is arranged between the second glass fibre bundle (3, 3', 3") and the optical system (1).

17. Endoscope according to claim 15, **characterised in that** a means (16, 17) for beam shaping is mounted on the distal end of the insufflation channel (14), the means being used to clean the distal end (1.2) of the endoscope and, in particular, the image entrance face.

18. Endoscope according to claim 1, **characterised in that** a heater (18) is provided in the jacket tube (19) to control the temperature of an insufflation gas conveyed in the insufflation channel (14).

19. Endoscope according to claim 1, **characterised in that** a heater (18) is provided in the jacket tube (19) to control the temperature of a flushing liquid conveyed in the flushing channel (15).

20. Endoscope according to claim 2, **characterised in that** a heater (18) is provided in the jacket tube (19) to control the temperature of the jacket tube (19), including at least one of the glass fibre bundles (2, 2', 3, 3', 3").

21. Endoscope according to claim 15, **characterised in that** a means (16, 17) for beam shaping is mounted on the distal end of the flushing channel (15), the means being used to clean the distal end (1.2) of the endoscope and, in particular, the image entrance face.

22. Endoscope according to claims 2 and 15, **characterised in that** the flushing channel (15) is arranged between the second glass fibre bundle (3, 3', 3") and the optical system (1).

## Revendications

1. Endoscope rigide constitué par un tube enveloppe (19) comportant une optique (1) et un premier faisceau de fibres de verre (2, 2'), qui émet parallèlement à l'axe d'image (4) de l'optique (1) et un second système optique d'éclairement, dans lequel le premier faisceau de fibres de verre (2, 2') et le second système optique d'éclairement sont disposés de telle sorte qu'ils éclairent un champ d'observation détecté par l'optique (1) et dans lequel la direction (3.1) de sortie de la lumière au niveau de la sortie de la lumière (3.2, 3.2', 3.2") du second système optique d'éclairement est décalée latéralement par rapport à la direction d'observation, conformément à l'axe d'image (4) sur l'extrémité distale (1.2) de l'optique (1), d'une distance telle qu'en raison de la parallaxe obtenue et de la formation ou de la distribution non uniforme de la lumière, ainsi obtenue, la plasticité de la reproduction de l'image dans le cas d'objets tridimensionnels (8) est accrue, **caractérisé en ce que** le second système optique d'éclairement est décalé d'une distance (d) dans la gamme comprise entre environ 5 et 50 mm, en retrait par rapport à l'extrémité distale (1.2) de l'optique (1).

2. Endoscope selon la revendication 1, **caractérisé qu'en ce** qui concerne le second système optique d'éclairement, il s'agit d'un faisceau de fibres de verre (3, 3', 3").

3. Endoscope selon la revendication 2, **caractérisé en ce que** les fibres de verre du premier et/ou du second faisceaux de fibres de verre (2, 2', 3, 3', 3") sont disposées en étant réparties sur une plage angulaire coaxialement autour de l'optique (1).

4. Endoscope selon la revendication 3, **caractérisé en ce que** le premier et/ou le second faisceau de fibres de verre (2, 2', 3, 3', 3") possèdent une section transversale en forme de croissant.

5. Endoscope selon la revendication 2, **caractérisé en ce que** la direction (3.1) de sortie de la lumière du second faisceau de fibres de verre (3, 3', 3") et la direction d'observation de l'optique (1) sont orientées en étant inclinées par rapport à l'axe longitudinal (1.5) du tube enveloppe (19).

6. Endoscope selon la revendication 1, **caractérisé en ce que** l'inclinaison de la direction (3.1) de sortie de la lumière du second faisceau de fibres de verre (3, 3', 3") et la direction d'observation de l'optique (1) s'étendent dans le même sens.

7. Endoscope selon les revendications 5 ou 6, **caractérisé en ce que** la direction (3.1) de sortie de la lumière du second faisceau de fibres de verre (3, 3', 3") fait avec la direction d'observation (4) de l'optique (1) un angle α (5) supérieur à zéro.

8. Endoscope selon la revendication 2, **caractérisé en ce que** le second faisceau de fibres de verre (3, 3', 3") est intégré dans le tube enveloppe (19).

9. Endoscope selon la revendication 2, **caractérisé en ce qu'**aussi bien les sorties de lumière (2.2, 3.2, 3.2', 3.2") des premier et second faisceaux de fibres de verre (2, 2', 3, 3', 3") ainsi que, également, la partie (1.1), située côté objet, de l'optique (1) sont disposées dans la zone de l'extrémité distale (1.2) de l'endoscope.

10. Endoscope selon la revendication 2, **caractérisé en ce que** les fibres de verre comportent, dans la sortie de lumière (3.2, 3.2', 3.2") du second faisceau de fibres de verre (3, 3', 3"), une surface frontale polie plane, orientée perpendiculairement à la direction longitudinale des fibres ou que les fibres sont polies avec une inclinaison sous un angle β (12) par rapport à une telle surface frontale.

11. Endoscope selon la revendication 2, **caractérisé en ce que** la sortie de lumière (3.2, 3.2', 3.2") du second faisceau de fibres de verre (3, 3', 3") présentant notamment un poli plat est disposée de manière à être déplaçable longitudinalement par rapport au tube enveloppe (19), afin d'influer sur la formation d'ombrage.

12. Endoscope selon la revendication 2, **caractérisé en ce que** les extrémités proximales des premier et second faisceaux de fibres de verre (2, 2', 3, 3', 3") sont réunies dans un raccord commun (13) d'un câble formant guide de lumière.

13. Endoscope selon la revendication 12, **caractérisé en ce que** le raccord commun (13) du câble formant guide de lumière comprend un dispositif (13.1), notamment un diaphragme, servant à régler la quantité de lumière qui est injectée dans les extrémités proximale des premier et second faisceaux de fibres de verre (2, 2', 3, 3', 3") et/ou servant à régler la répartition de la quantité de lumière injectée entre les premier et second faisceaux de fibres de verre (2, 2', 3, 3', 3").

14. Endoscope selon la revendication 2, notamment selon la revendication 12, **caractérisé en ce que** le nombre des différentes fibres de verre des premier et second faisceaux de fibres de verre (2, 2', 3, 3', 3") est adapté respectivement à la quantité de lumière à transmettre.

15. Endoscope selon la revendication 1, **caractérisé en ce qu'**au moins un canal d'insufflation (14) et/ou au moins un canal de balayage (15) sont prévus dans le tube enveloppe (19).

16. Endoscope selon les revendications 2 et 15, **caractérisé en ce que** le canal d'insufflation (14) est disposé entre le second faisceau de fibres de verre (3, 3', 3") et l'optique (1).

17. Endoscope selon la revendication 15, **caractérisé en ce que** sur l'extrémité distale du canal d'insufflation (14) est disposé un moyen (16, 17) de formation d'un jet, qui sert à nettoyer l'extrémité distale (1.2) de l'endoscope et notamment la surface d'entrée de l'image.

18. Endoscope selon la revendication 1, **caractérisé en ce que** dans le tube enveloppe (19) est prévu un dispositif de chauffage (18) servant à mettre en température un gaz d'insufflation entraîné dans le canal d'insufflation (14).

19. Endoscope selon la revendication 1, **caractérisé en ce que** dans le tube enveloppe (19) il est prévu un dispositif de chauffage (19) pour mettre en température un liquide de balayage entraîné dans le canal de balayage (15).

20. Endoscope selon la revendication 2, **caractérisé en ce que** dans le tube enveloppe (19) il est prévu un dispositif de chauffage (18) pour mettre en température le tube enveloppe (19), y compris au moins l'un des faisceaux de fibres de verre (2, 2', 3, 3', 3").

21. Endoscope selon la revendication 15, **caractérisé en ce que** sur l'extrémité distale du canal de balayage (15) est disposé un moyen (16, 17) de formation d'un jet, qui est utilisé pour nettoyer l'extrémité distale (1.2) de l'endoscope et notamment la surface d'entrée de l'image.

22. Endoscope selon les revendications 2 et 15, **caractérisé en ce que** le canal de balayage (15) est disposé entre le second faisceau de fibres de verre (3, 3', 3") et l'optique (1).
